(19) Europäisches Patentamt / European Patent Office / Office européen des brevets

(11) **EP 4 104 748 A1**

(12) **EUROPEAN PATENT APPLICATION**
published in accordance with Art. 153(4) EPC

(43) Date of publication:
**21.12.2022 Bulletin 2022/51**

(21) Application number: **20919086.7**

(22) Date of filing: **10.02.2020**

(51) International Patent Classification (IPC):
***A61B 3/028*** *(2006.01)*

(52) Cooperative Patent Classification (CPC):
**A61B 3/028**

(86) International application number:
**PCT/JP2020/005128**

(87) International publication number:
**WO 2021/161385 (19.08.2021 Gazette 2021/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SHIMADZU CORPORATION**
**Kyoto-shi, Kyoto 604-8511 (JP)**

(72) Inventor: **ARITA, Yoshiki**
**Kyoto-shi, Kyoto 604-8511 (JP)**

(74) Representative: **Müller-Boré & Partner**
**Patentanwälte PartG mbB**
**Friedenheimer Brücke 21**
**80639 München (DE)**

(54) **VISUAL FUNCTION TEST DEVICE**

(57)    A visual function test device 1 is provided with a housing 10 with a peephole; a visual target display 11 accommodated in the housing 10 to display a predetermined visual target; and virtual image optics accommodated in the housing 10, the virtual image optics being optics including lenses 121, 122 for forming a virtual image of the predetermined visual target at a position visible from the peephole, the virtual image optics having an exit pupil of a predetermined dimension. The visual function test device has an eye relief of 5 cm or more. By using the visual function test device 1, a visual function test can be performed without the need to mount equipment on a subject.

FIG. 2

## Description

## Technical Field

The present invention relates to a visual function test device.

## Background Art

In a visual acuity test, which is one of the visual function tests, conventionally, a method is employed in which a subject is made to visually recognize a predetermined visual target, such as, e.g., a Landolt ring, displayed on a visual acuity chart at a position away the visual acuity chart by a predetermined distance (usually 5 m) to measure the visual acuity. As a visual acuity chart, a visual acuity chart in which a predetermined visual target is printed as described in Non-Patent Document 1 and a visual acuity chart in which a predetermined visual target is changeably displayed on a liquid crystal screen as described in Non-Patent Document 2 are used. When a visual acuity test is performed using them, a visual target is visually recognized by a subject with an eye of the inspection target in a state in which the other eye on the non-inspection target side is covered with an occluder.

In the above-described visual acuity test, in order for the subject to visually recognize the visual target at a position away from the visual acuity chart by the predetermined distance, a space having a length at least equal to or larger than the predetermined distance is required. In order to enable a visual acuity test even in a smaller space, a vision meter has been proposed in which a visual target display unit for displaying a predetermined visual target and virtual image optics for forming a virtual image of the visual target at a position away from the visual target by the predetermined distance are provided. Non-Patent Document 3 describes a vision meter that displays a virtual image of a visual target inside a housing equipped with a forehead pad and two peepholes. In a visual acuity test using this visual acuity meter, the subject is made to visually recognize a virtual image of a visual target through a peephole corresponding to the eye of the inspection target in a state in which the forehead of the subject is fixed by being brought into contact with the forehead pad and the optical path of the eye on the non-inspection target side is blocked. In addition, Patent Document 1 and Non-Patent Documents 4 and 5 describe a visual acuity meter in which a virtual image of a visual target is displayed inside a housing having a visual target window formed on its front side. Non-Patent Document 4 describes that a visual target is displayed in a visually recognizable manner without missing any portion in a range of $\pm 70$ mm horizontally and $\pm 60$ mm vertically from the center of the visual target window, i.e., a visual target simultaneously visible with both eyes is displayed. Patent Document 1 describes that a lens unit that switches a lens in front of an eye at high speed is used to perform an examination per eye. Further, Non-Patent Document 5 describes that polarized glasses are used for performing an examination for each eye.

## Prior Art Documents

## Patent Document

**Patent Document 1:** Japanese Unexamined Patent Application Publication No. 2002-200041

## Non-Patent Document

**Non-Patent Document 1:** "LED visual acuity chart Milka II," [online], Inami & Co., Ltd., [Searched on November 19, 2019], Internet<URLhttp://inami.co.jp/files/topics/1358_ext_02_0.pdf>
**Non-Patent Document 2:** "Liquid Crystal visual acuity chart Systems Chart SC-1600," [online], Nidek Co., Ltd., [Searched on November 19, 2019], Internet<URL https://www.nidek.co.jp/products/ophthalmology/exami_list/exami_acuitychart/sc-1600.html>
**Non-Patent Document 3:** "Automatic Digital Vision NV-350," [online], NIDEC Co., Ltd., [Search on November 19, 2019], Internet<URL https://www.nidek.co.jp/products/glasses/optical_list/optical_acuitychart/nv-350.html>
**Non-Patent Document 4:** Tamai Hiromi, 10 others, "Utility of visual acuity chart Space Saving Chart (SSC-330 type II)," Japanese Orthoptic Journal 29, 121-125, 2001-11-30, Public Interest Corporation, Japan Society of Visual Trainees, Internet<URL https://www.jstage.jst.go.jp/article/jorthopticl977/29/0/29_0_121/_article/-char/ja/>
**Non-Patent Document 5:** "Space Saving Chart SSC-370 Type D," [online], Nidek Co., Ltd., [Searched on October 1, 2019], Internet<URL:https://www.nidek.co.jp/products/ophthalmology/exami_list/exami_acuitychart /ssc-370.html>
**Non-Patent Document 6:** "Clinical Guide", [online], Yamanaka Ophthalmologic Clinic, [Searched on November 19, 2019], Internet-URL: http://www.oputoyamanaka.com/guide/
**Non-Patent Document 7:** "3D Visual Function Trainer," [online], JFC Sales Plan Co., Ltd. [Searched on November 19, 2019], Internet<URL:http://www.jfcsp.co.jp/products/other-m/o-jfc/689>

## SUMMARY OF THE INVENTION

### Problems to be Solved by The Invention

It has been pointed out in Non-Patent Documents 1 and 2 that a visual acuity test is performed by blocking one eye that is not an inspection target with an occluder, but when a visual acuity test is performed by blocking one eye, the visual acuity adjusting function of the subject works unconsciously, which is likely to cause the test result to be near sight (e.g., Non-Patent Documents 5 and 6). Such issues do not arise with the visual acuity meters as described in Patent Document 1 and Non-Patent Documents 3 to 5. However, in the visual acuity meter as described in Non-Patent Document 3, since the visual acuity test is performed by bringing the forehead into contact with the forehead pad, it is likely to become unsanitary unless it is periodically disinfected or the like. In the visual acuity meter as described in Patent Document 1 and Non-Patent Documents 4 and 5, it takes time and labor to wear polarized glasses or shield eyes with a lens unit. In a case where the subject is a child, the child may be reluctant to wear such eyeglasses or the like.
Although the above is an example of performing a visual acuity test, the above-described visual acuity meter is used not only in a visual acuity test but also in a visual function test, such as, e.g., an ocular position test, an ocular movement test, a fundus test, a refractive power test, and the like (for example, Non-Patent Document 7). The same problems as described above arise when these visual function tests are performed.
An object of the present invention for solving the problems is to provide a visual function test device capable of performing a visual function test in a non-contact manner without the necessity of attaching equipment to a subject.

### Means for Solving the Problems

A visual function test device according to the present invention made to solve the above-described problems, is provided with:

a housing provided with a peephole;
a visual target display unit accommodated in the housing, the visual target display unit being configured to display a predetermined visual target; and
virtual image optics accommodated in the housing, the virtual image optics being optics including a lens for forming a virtual image of the predetermined visual target at a position visible from the peephole, the virtual image optics having an exit pupil of a predetermined dimension,
wherein the visual function test device has an eye relief of 5 cm or more.

### Effects of the Invention

The virtual image optics can be, for example, optics in which a convex lens is arranged such that the visual target display unit is positioned between the convex lens and its focal point. Alternatively, it may be configured such that an image of a visual target displayed on a visual target display unit is focused by one or more lenses (relay lenses) and the convex lens is positioned such that the image forming position of the visual target is positioned between the convex lens and its focal point.
The above-described predetermined dimension is set to be less than an interocular distance of a subject. The interocular distance differs depending on whether the subject is a child or an adult. Therefore, the above-described predetermined dimension is determined according to the subject for a visual function test.
The eye relief is conventionally known as an index indicating characteristics of a binocular. It specifically refers to the distance from the ocular lens of the binocular to the position furthest away from the ocular lens within the range in which the entire field of view can be visually recognized without missing any portion. A head-up display or a head-mounted display may not have an eyepiece lens (a lens paired with the objective lens). In such a case, the eye relief is broadly interpreted by the distance between the position of the eye in design and the optical element arranged at the position closest to the eye. In the same manner, the eye relief in the visual function test device according to the present invention means the distance from the position of the eye in design to the optical element arranged closest to the eye in the visual function test device, the distance being a distance at which the visual target displayed on the visual target display unit can be visually recognized without missing any part of the target. The eye relief according to the present invention is 5 cm or more. The length of the eye relief depends on the dimension of the visual target and/or the number of apertures of lens included in virtual image optics. The eye relief can be lengthened by making the visual target smaller or increasing the number of apertures of the lens.

In the visual function test device according to the present invention, since the dimension of the exit pupil of the virtual image optics is a predetermined dimension (less than the interocular distance of the subject), the virtual image cannot be visually recognized with the eye on the non-inspection target side. Therefore, there is no need to attach equipment to the subject. Further, since the eye relief is 5 cm or more and the virtual image of the visual target can be visually recognized at a position away from the visual function test device by 5 cm or more, the visual function can be examined in a non-contact manner.

**BRIEF DESCRIPTION OF THE DRAWINGS**

FIG. 1 is an external view of an example of a visual function test device according to the present invention.
FIG. 2 is a diagram showing the configuration of the inside of the housing of the visual function test device according to this example.
FIG. 3 is a diagram schematically showing features of the visual function test device according to this example.
FIG. 4 is a diagram schematically showing dimensions of an eye relief and an eye motion box in a conventional vision meter.
FIG. 5 is another diagram schematically showing dimensions of an eye relief and an eye motion box in a conventional vision meter.
FIG. 6 is a diagram illustrating a situation in which a louver is used in the visual function test device according to the example.
FIG. 7 is a diagram illustrating a situation in which a liquid crystal display provided with a backlight having directivity is used in the visual function test device according to this example.
FIG. 8 is a diagram illustrating a situation in which a microlens array is used in the visual function test device according to the example.
FIG. 9 is a diagram illustrating the configuration of the inside of the housing of the visual function test device according to a modification.

**EMBODIMENTS FOR CARRYING OUT THE INVENTION**

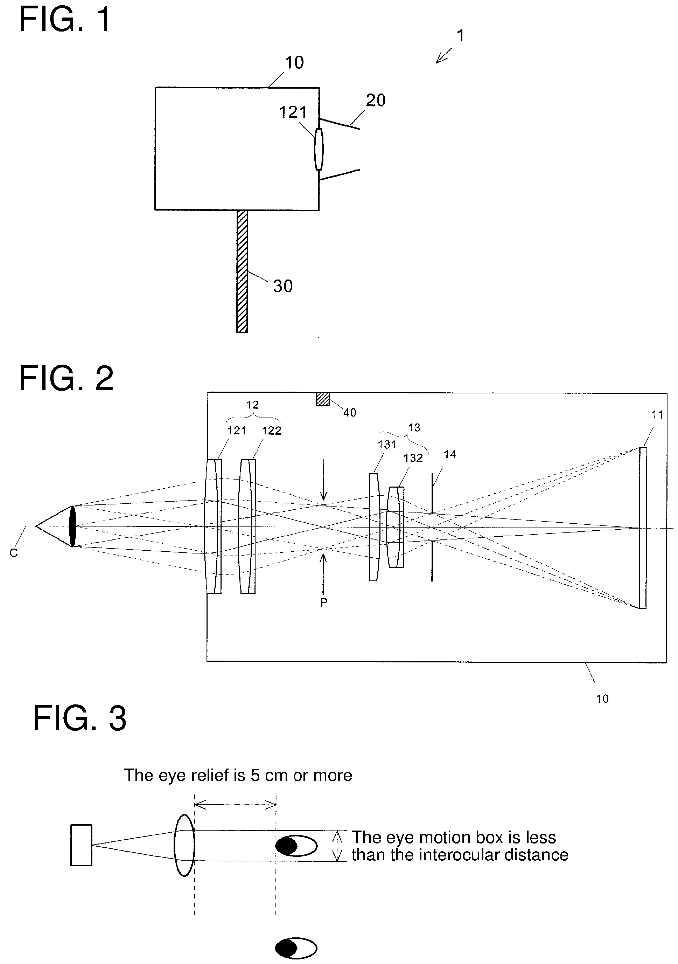

An example of a visual function test device according to the present invention will be described below with reference to the attached drawings. FIG. 1 is a cross-sectional view of a visual function test device 1 according to the example. Note that the illustration of the configuration inside the housing 10 is omitted. For the configuration inside the housing 10, please see FIG. 2.

The visual function test device 1 of this example is provided with: a housing 10; a light-shielding portion 20 attached to the front side (subject side) of the housing 10; and a support rod 30 provided on a lower surface of the housing 10. A peephole (window) is provided on the front side of the housing 10. A cylindrical light-shielding portion 20 having a configuration obtained by cutting off a top of a cone is attached to surround the peephole. A lens 121 having a predetermined number of apertures is attached to the peephole. The visual function test device 1 of this example is designed to perform a predetermined visual function test by making a subject grip the support rod 30 to visually recognize the virtual image of the visual target formed inside the housing 10 with the eye on the inspection target side from a position away from the lens 121 by a predetermined distance (e.g., 5 cm). One example of the visual function test is a visual acuity test. In this case, the subject is made to visually recognize, for example, a Landolt ring.

Inside the housing 10, a visual target display unit for displaying a predetermined visual target and virtual image optics having an exit pupil of a predetermined dimension are accommodated. The virtual image optics is optics including lenses that form a virtual image of the predetermined visual target at a position visible from the peephole. In order to suppress the generation of stray light in the housing 10 and make optical elements, etc., in the housing 10 difficult to be seen by the subject, the inner wall surface of the housing 10 is painted black. The visual target display unit of this example is a liquid crystal display 11 of a backlight system. The liquid crystal display 11 displays the predetermined visual target under control by a control unit (not shown),

FIG. 2 shows the arrangement of the liquid crystal display 11 and the virtual image optics arranged within the housing 10. A camera 40 shown in FIG. 2 is used as one preferred embodiment described below. The virtual image optics include, in order from the side closest to the peephole, a first lens set 12 and a second lens set 13. The first lens set 12 includes a first achromatic lens 121 and a second achromatic lens 122 in order from the side closest to the peephole. The second lens set 13 includes a plano-convex lens 131 and an achromatic lens 132 in order from the side closest to the peephole. Note that this plano-convex lens is merely an example. A biconvex lens, a biconcave lens, and a concavo-convex lens may be used by appropriately combining them. Further, between the achromatic lens 132 and the liquid crystal display 11, a diaphragm 14 is provided. These achromatic lenses are a combination of a negative lens and a positive lens composed of members having refractive indices differing from each other, and has a function of removing chromatic aberration, thereby enhancing imaging performance. Note that a negative lens (concave lens) refers to a lens that is thinner at the center than at the periphery. In a negative lens, light parallel to the optical axis is refracted and spread out by the lens. At this time, the diffused light spreads as if it were emitted from one point on the optical axis. A positive lens refers to a lens that is thicker at the center than at the edge. In a positive lens, light parallel to the optical axis (the line passing perpendicular to the lens at the center of the lens curved surface) is refracted by the lens and collected at one point on the optical axis.

In FIG. 2, the optical path of the light emitted from the center of the liquid crystal display 11, the optical path of the light emitted from the upper end, and the optical path of the light emitted from the lower end are shown by a solid line, a broken line, and a one-dot chain line, respectively. As shown by these optical paths, out of the light emitted from the liquid crystal display 11, only the light that has passed through the diaphragm 14 passes through the achromatic lens 132 and the plano-convex lens 131 of the second lens set 13 in order. Thereby, the image of the visual target displayed on the liquid crystal display 11 is formed at the image forming position P. That is, the second lens set 13 constitutes imaging optics. The light flux emitted from the liquid crystal display 11 propagates in a direction of the central axis by the optical axis C and is incident on the eye of the subject.

The first achromatic lens 121 and the second achromatic lens 122 of the first lens set 12 are positioned so that their focal points are located farther away from the position of the eye of the subject at the time of the visual function test. For this reason, the virtual image of the image of the index imaged at the image forming position P appears at a position away from the eye of the subject by a certain distance (e.g., 5 m in the case of a visual acuity test). That is, the first lens set 12 constitutes virtual image forming optics.

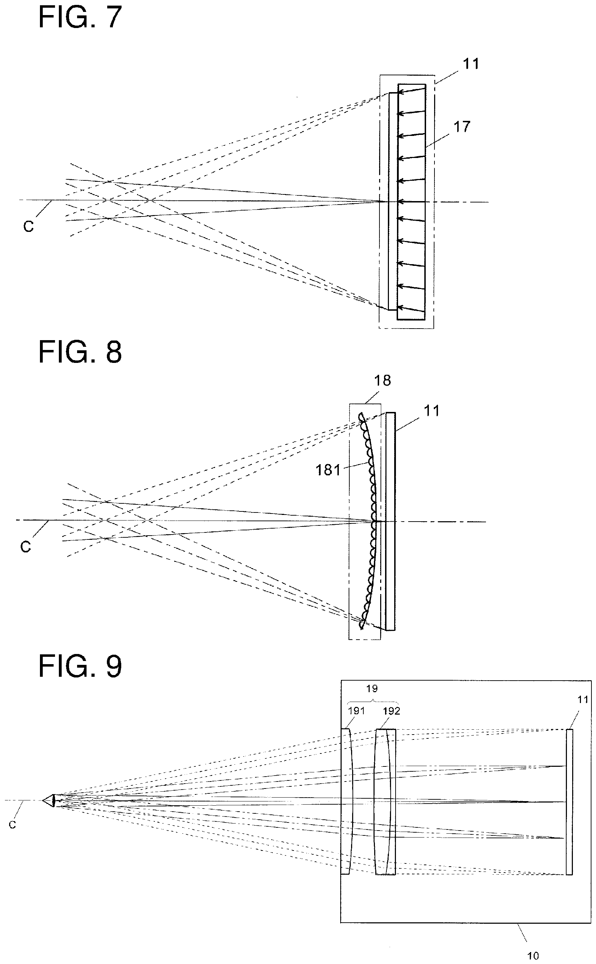

In the visual function test device 1 of this example, as schematically shown in FIG. 3, it is designed such that the dimension of the exit pupil is a predetermined dimension. The predetermined dimension denotes a dimension less than the interocular distance of the subject. Adults have an interocular distance of about 7 cm. Therefore, in a case where it is used for the vision measurement of an adult subject, the dimension of the exit pupil is set to be less than 7 cm. On the other hand, the child's interocular distance is shorter than that. Thus, in a case where it is used for a measurement of visual acuity for a child subject, the predetermined dimension is set to be less than its interocular distance. In the visual function test device 1 of this example, the dimension of the exit pupil corresponds to the dimension of the light flux (In FIG. 2, it is illustrated as the dimension of the eye; the same is applied to FIG. 9 to be described later) at the position of the eye of the subject.

Here, the design of the visual function test device 1 will now be described by way of examples. In a case where the field of view (actual field of view) determined by the dimension of the visual target to be displayed is φ6 (6 degrees), the dimension of the exit pupil is 30 mm (less than the interocular distance of the subject), and the eye relief is 200 mm, the dimension M of the optical device arranged closest to the eye can be calculated from the following equation.

$$M \text{ (the dimension of the optical element)} = 30 \text{ mm (the dimension of the exit pupil}$$

$$\text{dimension)} + 2 \times 200 \text{ mm (the eye relief)} \times \tan (6 \text{ degrees (the dimension of the visual field)} /$$

$$2) \fallingdotseq 51 \text{ mm} \quad ... (1)$$

In the visual function test device 1 of this example, as described above, since the dimension of the exit pupil is less than the interocular distance of the subject, for example, when examining the right eye, the virtual image is not visually recognized by the left eye (the eye on the non-inspection target side) when the virtual image of the index is visually recognized by the right eye (the eye on the non-inspection target side).

Further, in the visual function test device 1 of this example, as schematically shown in FIG. 3, the eye relief is 5 cm or more. The eye relief is conventionally known as an indicator index characteristics of a binocular. It specifically refers to the distance from the ocular lens of the binocular to the position farthest away from the ocular lens within the range in which the entire field of view can be visually recognized without missing any portion of the field of view. Further, a head-up or a head-mounted display may not have an eyepiece lens (a lens paired with the objective lens). In such a case, the eye relief is broadly interpreted by the distance between the position of the eye in design and the optical element arranged at the position closest to the eye. In the same manner, the eye relief according to this example means the distance from the position of the eye in design to the optical element (first achromatic lens 121) arranged closest to the eye in the visual function test device 1. The eye relief can be lengthened by increasing the number of apertures of the lens 121 attached to the peephole or by making (narrowing the field of view) the visual target to be displayed on the liquid crystal display 11 smaller. The eye relief may be appropriately determined as long as it is 5 cm or more, and may be, for example, 30 cm or more. This suppresses the involvement of subject's unconscious adjustment of the visual acuity, enabling an accurate examination.

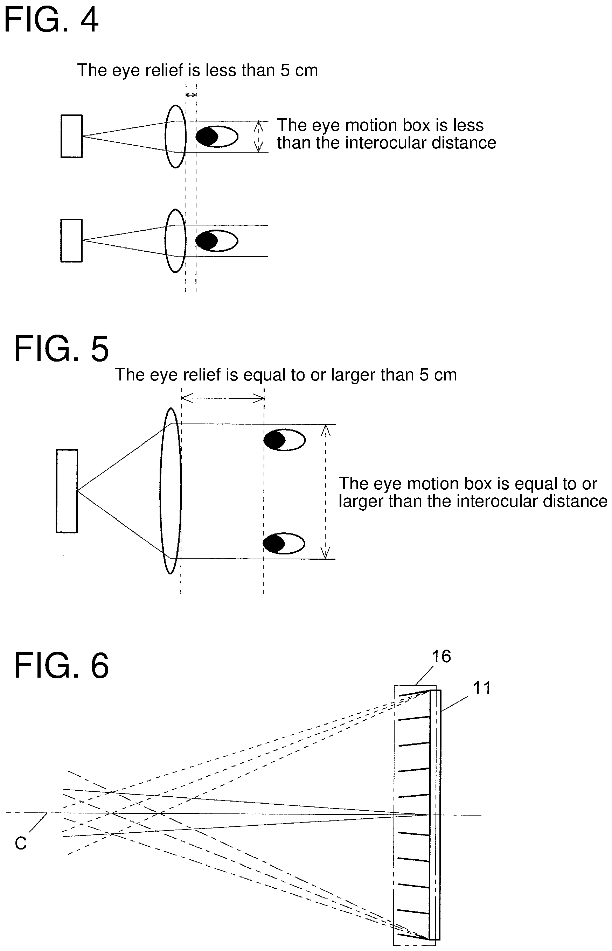

FIGS. 4 and 5 schematically illustrate the dimension of the eye relief and the dimension of the exit pupil (i.e., the dimension of the eye motion box) of a conventional vision meter. FIG. 4 relates to a visual acuity meter as disclosed by Non-Patent Document 3. In this dynamometer, a virtual image of a visual target is displayed in a housing provided with a forehead pad and two peepholes. In a state in which the forehead of the subject is in contact with the forehead pad, the distance between the eyepiece lens attached to the peephole and the eye of the subject is about 3 cm, and therefore, the dimension of the eye relief is set to the same level. In such a vision meter, a visual acuity test is carried out with the forehead of the subject in contact with the forehead pad, and therefore, it is likely to become unsanitary unless it is periodically disinfected or the like.

FIG. 5 shows a visual acuity meter as described by Patent Document 1 and Non-Patent Documents 4 and 5. In this vision meter, the virtual image of the visual target is displayed inside the housing with a visual target window formed in the front surface. As described in Non-Patent Document 4, the visual target can be visually recognized in the range of ±70 mm horizontally and ±60 mm vertically from the center of the visual target window without missing. In other words, the dimension of the exit pupil (i.e., the dimension of the eye motion box) is equal to or larger than the interocular distance. In such a visual acuity meter, in the case of performing an examination on an eye-by-eye basis, it is required to wear polarizing glasses or the like. In a case where a subject is a child, the child may be reluctant to wear such glasses.

On the other hand, in the visual function test device 1 of this example, since the dimension of the exit pupil of the virtual image optics is a predetermined dimension (less than the interocular distance of the subject), the virtual image cannot be visually recognized by the eye on the non-inspection target side. Therefore, there is no need for the subject to wear equipment. Further, since the eye relief is 5 cm or more and the virtual image of the visual target can be visually recognized at a position away from the visual function test device 1 by 5 cm or more, the visual function can be examined in a non-contact manner.

The above-described example is one example and can be appropriately modified in accordance with the spirit of the present invention. In the visual function test device 1 of the above-described example, although it is configured to limit the dimension of the exit pupil by the diaphragm 14, as shown in FIG. 6, a louver 16 may be attached on the front surface of the liquid crystal display 11. The louver 16 is arranged such that the light parallel to the optical axis C is allowed to pass at the central portion of the liquid crystal display 11 and the inclination with respect to the optical axis C increases as it approaches the the end of the liquid crystal display 11. Thus, it is possible to limit the light flux emitted from the liquid crystal display 11 in the same manner as when the diaphragm 14 is provided.

Alternatively, as shown in FIG. 7, a backlight of the liquid crystal display 11 having directivity may be used. In this case, a backlight 17 may be used in which light is emitted in a direction parallel to the optical axis C at the central portion of the liquid crystal display 11, and light is emitted in a direction in which the incline with respect to the optical axis C increases as it advances toward the end of the liquid crystal display 11. By using such a backlight 17, it is possible to limit the light flux emitted from the liquid crystal display 11 in the same manner as when providing the diaphragm 14.

Alternatively, as shown in FIG. 8, a microlens array 18 may be attached on the front side of the liquid crystal display 11. The microlens array 18 is configured by a number of tiny lenses 181 arranged such that at the center of the liquid crystal display 11, the top of the lens 181 faces in the direction parallel to the optical axis C, and the top of the lens 181 faces toward the direction inclined with respect to the optical axis C as it advances toward the end of the liquid crystal display 11. By providing directivity to the light passing through the respective lenses 181 arranged as described above, it is possible to limit the light flux emitted from the liquid crystal display 11 in the same manner as in the case of providing the diaphragm 14. FIG. 8 shows that micro lenses 181 having the same shape are arranged on a curved base. However, it is also possible to use lenses having different shapes on a flat base (the top of each micro lens 181 is processed such that the top of a lens 181 faces in a direction parallel to the optical axis C at the center of the liquid crystal display 11 and the top of a lens 181 faces toward the direction inclined with respect to the optical axis C of the liquid crystal display 11 as it advances toward the end of the liquid crystal display 11).

Although FIG. 2 shows an example in which the optical axis C from the liquid crystal display 11 to the subject's eye is a straight line, a mirror may be arranged at an appropriate position and the optical axis C may be bent. This allows a compact configuration without increasing the size of the housing (eye motion box) 10 in a particular one direction. Alternatively, the mirror may be reflective optics (including a concave mirror and a convex mirror) with a powered mirror instead of a lens. Furthermore, reflective refractive index optics which is a combination of a powered mirror and a lens may be used.

Further, in the visual function test device 1 of the above-described example, it is possible to change the position of the optical elements and the liquid crystal display 11. For example, by changing the position of the second lens set 13 of the virtual image optics, the image forming position P of the index may be moved, thereby changing the position (subject's eye) at which the virtual image of the visual target is formed. Alternatively, by changing the position of the liquid crystal display 11, the image forming position P of the index may be moved, thereby changing the position at which the virtual image of the visual target is formed (the distance from subject's eye). By adopting such a configuration, it is possible to perform a near-mid-range examination, a near-point distance examination of presbyopia, or the like.

Further, in the visual function test device 1 of the above-described example, it is configured such that the subject is made to grip the support rod 30 to visually recognize the virtual image of the visual target formed inside the housing 10 supported by the support rod 30 by one eye of the subject, but instead, the housing 10 may be placed on a desk. Further, it may be configured such that one housing 10 is provided with two peepholes each having a lens 121, and virtual image optics as described with reference to FIG. 2 may be arranged at a position corresponding to each peephole. This makes it possible to perform a visual function test of not only one eye but also both eyes.

Further, in the visual function test device 1 of the above-described example, it may be configured such that a camera 40 is arranged inside the housing 10 to image the condition of the eye (eye position, eye movement, fundus, refractive index, etc.) of the subject who visually recognizes the visual target by the camera to perform the examination.

The above-described examples are preferable examples of a visual function test device, and a part of constituent components (optical elements, etc.) included in the visual function test device 1 of the examples may be omitted. As shown in FIG. 9 as a modification, only the liquid crystal display 11 and the lens set 19 (the plano-convex lens 191 and the achromatic lens 192) forming the virtual image of the visual target displayed on the liquid crystal display 11 are arranged in the housing 10, and the plano-convex lens 191 is arranged at the peephole of the housing. By adopting such a simple configuration, the device can be miniaturized and manufactured inexpensively. Since this configuration does not include a diaphragm, in order to limit the dimension of the exit pupil, the light flux emitted from the display 11 may be limited by combining the configurations described with reference to FIGS. 6 to 8.

In the above-described examples, a visual target is displayed on the liquid crystal display 11. However, in addition to the configuration, any configuration may be used in which a visual target is illuminated from the back by placing a sheet preprinted with a plurality of visual targets, and the visual target to be illuminated can be arranged in a switchable manner. Further, in the above-described example, a cylindrical light-shielding portion 20 is arranged so as to surround the peephole. However, as long as the light-shielding portion 20 prevents the visual target from being visually recognized by the eye on the non-inspection target side (blocks the line of sight from the eye on the noon-inspection target side toward the peephole side), an appropriate shape may be used by arranging it at an appropriate position. Further, as long as it is possible to sufficiently shield unwanted light rays by optics, the light-shielding portion 20 may be omitted. Further, although lenses 121 and 191 are attached to the peephole in the above-described examples, these lenses 121 and 191 may be accommodated inside the housing 10, and the peephole may be opened, or a transparent plate member (a transparent member which is not an optical element) may be arranged in the peephole.

**[Aspects of the Invention]**

It will be understood by those skilled in the art that the plurality of exemplary embodiments described above is illustrative of the following aspects.

**(Item 1)**

A visual function test device according to one aspect of the present invention includes:

a housing provided with a peephole;
a visual target display unit accommodated in the housing, the visual target display unit being configured to display a predetermined visual target; and
virtual image optics accommodated in the housing, the virtual image optics being optics including a lens for forming a virtual image of the predetermined visual target at a position visible from the peephole, the virtual image optics having an exit pupil of a predetermined dimension,
wherein the visual function test device has an eye relief of 5 cm or more.

In the visual function test device as recited in the above-described Item [1], the predetermined dimension is less than the interocular distance of the subject. The interocular distance differs depending on whether the subject is a child or an adult. Therefore, the predetermined dimension is determined according to the subject in a visual function test.

In the visual function test device as recited in the above-described Item [1], since the dimension of the exit pupil of the virtual image optics is a predetermined dimension (less than the interocular distance of the subject), the virtual image is not visually recognized by the eye on the non-inspection target side. Therefore, there is no need to attach equipment to the subject. In addition, since the eye relief is 5 cm or more and the virtual image of the visual target can be visually recognized at a position away from the visual function test device by 5 cm or more, the visual function can be examined in a non-contact manner.

7

**(Item 2)**

In the visual function test device as recited in the above-described Item [1], the virtual image optics is provided with imaging optics configured to form an image of the visual target displayed on the visual target display unit between the target display unit and the lens.

In the visual function test device as recited in the above-described Item [2], by forming the image of the visual target at a position closer to the visual target display unit, it is possible to make the visual target difficult to be seen by the eye on the non-inspection target side.

**(Item 3)**

In the visual function test device as recited in the above-described Item [2], the imaging optics has a diaphragm.

In the visual function test device as recited in the above-described Item [3], the eye relief can be adjusted by appropriately changing the dimension of the field of view by the diaphragm.

**(Item 4)**

In the visual function test device as recited in any one of the above-described Items [1] to [3], the visual function test device is further provided with:

a directivity imparting part configured to impart directivity to a light flux from the visual target display unit to the peephole.

In the visual function test device as recited in the above-described Item [4], the directivity imparting part restricts the direction of the light from the visual target display unit, thereby making the visual target less visible to the non-inspection target eye.

**(Item 5)**

In the visual function test device as recited in the above-described Item [4], the directivity imparting part is a louver attached to the visual target display device.

In the visual function test device as recited in the above-described Item [5], it can be configured easily and inexpensively by simply attaching a louver to the visual target display device.

**(Item 6)**

In the visual function test device as recited in the above-described Item [4],

the visual target display device is a liquid crystal display, and
the directivity imparting part is a backlight provided on the liquid crystal display, the backlight being configured to emit light having directivity.

In the visual function test device as recited in the above-described Item [6], it utilizes the liquid crystal display's backlight as a directivity imparting part, which makes it difficult to visually recognize the visual target with the eye on the non-inspection target side without increasing the number of components.

**(Item 7)**

In the visual function test device as recited in the above-described Item [4], the directivity imparting part is a microlens array arranged between the visual target display device and the peeping window.

In the visual function test device as recited in the above-described Item [7] directivity can be accurately given to the light flux by appropriately designing the microarray lens.

**(Item 8)**

In the visual function test device as recited in any one of the above-described Items [1] to [7], it further includes:

a light-shielding portion mounted around the peephole, the light-shielding portion being configured to block a part of the light emitted from the peephole.

In the visual function test device as recited in the above-described Item [8], the light-shielding portion can reliably prevent the visual target from being seen by the eye on the non-inspection target side.

**(Item 9)**

In the visual function test device as recited in any one of the above-described Items [1] to [8], it further includes: an imaging unit configured to track a line of sight of a subject visually recognizing the predetermined index through the peephole.

In the visual function test device as recited in the above-described Item [9], by tracking the line of sight of the subject with the imaging unit (e.g., camera), various examinations, such as, e.g., an eye position, an eye movement, a fundus oculi, and a refractive index, can be performed.

**(Item 10)**

In the visual function test device as recited in any one of the above-described items [1] to [8], the virtual image optics is capable of changing a position where the virtual image of the predetermined visual target is formed.

In the visual function test device as recited in the above-described Item [10], by changing the position where the virtual image of the visual target is formed, it is possible to perform a far-to-near examination, a presbyopia near-point distance examination, or the like.

**Description of Reference Symbols**

| | |
|---|---|
| 1: | Visual function test device |
| 10: | Housing |
| 11: | Liquid crystal display |
| 12: | First lens set |

| | |
|---|---|
| 121: | First achromatic lens |
| 122: | Second achromatic lens |

| | |
|---|---|
| 13: | Second lens set |

| | |
|---|---|
| 131: | Plano-convex lens |
| 132: | Achromatic lens |

| | |
|---|---|
| 14: | Diaphragm |
| 16: | Louver |
| 17: | Backlight |
| 18: | Microlens array |

| | |
|---|---|
| 181: | Lens |

| | |
|---|---|
| 19: | Lens set |

| | |
|---|---|
| 191: | Plano-convex lens |
| 192: | Achromatic lens |

| | |
|---|---|
| 20: | Light-shielding portion |
| 30: | Support rod |
| 40: | Camera |
| C: | Optical axis |
| P: | Image forming position |

**Claims**

1. A visual function test device comprising:

a housing provided with a peephole;

EP 4 104 748 A1

a visual target display unit accommodated in the housing, the visual target display unit being configured to display a predetermined visual target; and
virtual image optics accommodated in the housing, the virtual image optics being optics including a lens for forming a virtual image of the predetermined visual target at a position visible from the peephole, the virtual image optics having an exit pupil of a predetermined dimension,
wherein the visual function test device has an eye relief of 5 cm or more.

2. The visual function test device as recited in claim 1,
wherein the virtual image optics is provided with imaging optics configured to form an image of the visual target displayed on the visual target display unit between the target display unit and the lens.

3. The visual function test device as recited in claim 2,
wherein the imaging optics has a diaphragm.

4. The visual function test device as recited in claim 1, further comprising:
a directivity imparting part configured to impart directivity to a light flux from the visual target display unit to the peephole.

5. The visual function test device as recited in claim 4,
wherein the directivity imparting part is a louver attached to the visual target display device.

6. The visual function test device as recited in claim 4,

wherein the visual target display device is a liquid crystal display, and
wherein the directivity imparting part is a backlight provided on the liquid crystal display, the backlight being configured to emit light having directivity.

7. The visual function test device as recited in claim 4,
wherein the directivity imparting part is a microlens array arranged between the visual target display device and the peeping window.

8. The visual function test device as recited in claim 1, further comprising:
a light-shielding portion mounted around the peephole, the light-shielding portion being configured to block a part of the light emitted from the peephole.

9. The visual function test device as recited in claim 1, further comprising:
an imaging unit configured to track a line of sight of a subject visually recognizing the predetermined index through the peephole.

10. The visual function test device as recited in claim 1,
wherein the virtual image optics is capable of changing a position where the virtual image of the predetermined visual target is formed.

# FIG. 1

10

121

20

1

30

# FIG. 2

12

121 122

40

13

131

132

14

11

C

P

10

# FIG. 3

The eye relief is 5 cm or more

The eye motion box is less
than the interocular distance

# FIG. 4

The eye relief is less than 5 cm

The eye motion box is less than the interocular distance

# FIG. 5

The eye relief is equal to or larger than 5 cm

The eye motion box is equal to or larger than the interocular distance

# FIG. 6

16

11

C

FIG. 7

FIG. 8

FIG. 9

**EP 4 104 748 A1**

<table>
<tr><td colspan="2" style="text-align:center">INTERNATIONAL SEARCH REPORT</td><td colspan="2">International application No.<br><br>PCT/JP2020/005128</td></tr>
</table>

**A. CLASSIFICATION OF SUBJECT MATTER**
Int.Cl. A61B3/028(2006.01)i
FI: A61B3/028

According to International Patent Classification (IPC) or to both national classification and IPC

**B. FIELDS SEARCHED**

Minimum documentation searched (classification system followed by classification symbols)
Int.Cl. A61B3/028

Documentation searched other than minimum documentation to the extent that such documents are included in the fields searched
Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 58-89235 A (ESSILOR INTERNATIONAL COMPAGNIE GENERALE D' OPTIQUE) 27.05.1983 (1983-05-27), page 2, lower left column, lines 2-7, page 3, lower right column, line 19, page 4, upper right column, line 15 to page 4, lower left column, line 1, page 4, lower right column, lines 4-20, page 5, lower left column, lines 5-10, page 5, lower right column, lines 9-18, page 6, upper right column, lines 6-20, fig. 1-3, 6, 7 | 1, 10<br>2-3, 8-9<br>4-7 |
| Y<br>A | JP 58-99945 A (CANON INC.) 14.06.1983 (1983-06-14), page 4, lower left column, lines 11-15 | 2-3<br>4-7 |
| Y<br>A | JP 2015-500732 A (POSTECH ACADEMY-INDUSTRY FOUNDATION) 08.01.2015 (2015-01-08), paragraph [0035], fig. 2 | 8<br>4-7 |

☒ Further documents are listed in the continuation of Box C.  ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>03.03.2020 | Date of mailing of the international search report<br>17.03.2020 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

| International application No. |
| --- |
| PCT/JP2020/005128 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y<br>A | JP 2009-273869 A (PANASONIC ELECTRIC WORKS CO., LTD.) 26.11.2009 (2009-11-26), paragraph [0074] | 9<br>4-7 |
| A | US 6042231 A (VEGA VISTA, INC.) 28.03.2000 (2000-03-28), fig. 2(a) | 1-10 |

Form PCT/ISA/210 (second sheet) (January 2015)

**INTERNATIONAL SEARCH REPORT**
Information on patent family members

International application No.

PCT/JP2020/005128

| | | |
|---|---|---|
| JP 58-89235 A | 27.05.1983 | US 4572629 A<br>column 1, lines 5-8, column 3, line 21,<br>column 3, lines 50-56,<br>column 4, lines 7-25, column 4,<br>line 64 to column 5, line 2,<br>column 5, lines 21-32, column 5,<br>line 62 to column 6, line 4,<br>fig. 1-3, 6, 7 |
| JP 58-99945 A | 14.06.1983 | (Family: none) |
| JP 2015-500732 A | 08.01.2015 | US 2014/0340642 A1<br>paragraph [0053], fig. 2 |
| JP 2009-273869 A | 26.11.2009 | US 2011/0205491 A1<br>paragraph [0085] |
| US 6042231 A | 28.03.2000 | WO 98/05251 A1 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002200041 A

**Non-patent literature cited in the description**

- LED visual acuity chart Milka II. Inami & Co., Ltd, 19 November 2019
- Liquid Crystal visual acuity chart Systems Chart SC-1600. Nidek Co., Ltd, 19 November 2019
- Automatic Digital Vision NV-350. NIDEC Co., Ltd, 19 November 2019
- Utility of visual acuity chart Space Saving Chart (SSC-330 type II). **TAMAI HIROMI.** Japanese Orthoptic Journal. Interest Corporation, Japan Society of Visual Trainees, 30 November 2001, vol. 29, 121-125
- Space Saving Chart SSC-370 Type D. Nidek Co., Ltd, 01 October 2019
- Clinical Guide. Yamanaka Ophthalmologic Clinic, 19 November 2019
- 3D Visual Function Trainer. JFC Sales Plan Co., Ltd, 19 November 2019